Europäisches Patentamt

European Patent Office

Office européen des brevets

(11) Publication number: **0 415 565 A2**

# EUROPEAN PATENT APPLICATION

(21) Application number: 90308408.5

(22) Date of filing: 31.07.90

(51) Int. Cl.⁵: **C12N 1/16,** C12N 1/38, C12P 21/02, C07K 13/00, C12N 15/81

(30) Priority: 31.07.89 US 387679

(43) Date of publication of application: 06.03.91 Bulletin 91/10

(84) Designated Contracting States: BE CH DE FR GB IT LI NL

(71) Applicant: MERCK & CO. INC. 126, East Lincoln Avenue P.O. Box 2000 Rahway New Jersey 07065-0900(US)

(72) Inventor: Schulman, Cheryl A. 84 Pine Valley Road Doylestown, PA 18901(US)

(74) Representative: Hesketh, Alan, Dr. et al European Patent Department Merck & Co., Inc. Terlings Park Eastwick Road Harlow Essex, CM20 2QR(GB)

(54) **Enhanced expression of heterologous proteins in recombinant hosts in a non-growth media.**

(57) The present invention relates to a fermentation process for producing recombinant proteins in a recombinant host strain. The process can be adapted for organisms containing a mutation in cell wall and/or plasma membrane biosynthesis (e.g. mnn9 ) in a non-growth media. An example of such a strain is described in Merck's Application serial number 202,764, filed June 3, 1988, corresponding to EP-A-0344864. Additionally, the process can be adapted for organisms containing "wild-type" cell walls and plasma membrane.

EP 0 415 565 A2

# ENHANCED EXPRESSION OF HETEROLOGOUS PROTEINS IN RECOMBINANT HOSTS IN A NON-GROWTH MEDIA

## BACKGROUND OF THE INVENTION

Production of recombinant proteins requires, in part, a promoter, coding region for the protein, and a terminator. The promoter drives the expression of the protein and the terminator is the signal to stop expression. Some promoters (regulatable) require removal of a compound (repressor, e.g. inorganic phosphate for PH05 the promoter) or a change in environmental conditions (e.g temperature change for the $\alpha$-mating factor promoter) to be activated.

Typically, compounds are added to the medium (e.g galactose) or depleted from the medium during normal growth (e.g. phosphate) or environmental conditions are altered (e.g. temperature shift) such that expression of the heterologous protein can be initiated.

Occasionally, however, the medium in which the cells are growing interfers with the expression of heterologous proteins due to the presence of inhibiting substances. In such cases, cells are typically harvested in late log to early stationary phase and separated from the growth medium that contains the inhibitory substances. The cells are then transferred to a second growth medium that lacks the inhibitory substances and promotes the expression of the heterologous protein. Examples of this method of inducing expression of heterologous proteins have been described in European Patent Application 86401710.8.

One of the challenges of isolating recombinant proteins is separating the desired protein from the extraneous proteins in either the fermentation broth (for secreted products) or in the cellular extract (for intracellular products). Isolating proteins that are secreted from the cells is often complicated by growth media that are rich in nutrients, as would be expected from, for example, a fed-batch fermentation process. Isolating intracellular proteins might also be complicated if the cells are grown in a nutrient-rich medium and contain large pools of reserve proteins and metabolites.

Various types of host cells can be used for the production of heterologous proteins including, but not limited to, Escherichia coli , Bacillus sp., Saccharomyces cerevisiae , mammalian cells, and insect cells. Aside from specific nutrient requirements or special medium additions to select for cells containing the recombinant vector (e.g. antibiotic supplementation), some cells require special conditions for growth and/or maintenance of the integrity of the cells. Non-inclusive examples of cell types that require special conditions for the growth and/or maintenance of the cells are mammalian cells and yeast containing mutations in cell wall or membrane biosynthesis. A non-inclusive example of a mutation in the biosynthesis of cell membranes is the mnn9 mutation of S . cerevisiae.

The description of the genotype of the mnn9 gene of S. cerevisiae was described by Ballou et al . (1980 Saccharomyces cerevisiae mutants that make mannoproteins with a truncated carbohydrate outer chain. J. Biol. Chem. 255 :5986-5991) and Tsai et al . (1984 Carbohydrate structure of Saccharomyces cerevisiae mnn9 mannoprotein. J. Biol. Chem. 260 :3805-3811). Strains of yeast with the mnn9 genotype are unable to glycosylate proteins beyond the core mannan backbone (please see Gopal and Ballou. PNAS. 84 :8824(1987)). Cells containing the mnn9 mutation are grown typically in YEPD medium (1% yeast extract/2% Bacto-peptone/2% D-glucose) as described in J. Biol. Chem. 255 :5986-5991; the cells generated from these fermentations have been used to study the biochemistry of yeast glycosylation. Occasionally, revertant MNN9 strains would overgrow the mnn9 cells in YEPD medium.

One consequence of the mnn9 mutation on the yeast is an increased fragility of the cell as described in Ballou (1980. J. Biol. Chem. 255 :5986-5991). It has been suggested that this fragility can be compensated for by adding sorbitol to the medium. It is obvious to those skilled in the art that the invention described herein extends to other mutations in cell wall and/or membrane biosynthesis other than mnn9 .

## SUMMARY OF THE INVENTION

The present invention relates to a medium as well as a process for the production of recombinant proteins in a non-growth medium. In the case of cells which exhibit fragility to osmotic stress, the invention includes optimizing the osmotic stability of the medium and maximizing productivity of heterologous proteins. More specifically, the present invention relates to a medium with an assimilable source of nitrogen, amino acids, and an assimilable carbon source whose osmolarity may be adjusted either through increased concentrations of medium components or by the addition of an extraneous osmotic stabilizer for growth of the cells and then transferring these cells to a second medium for the production of the heterologous protein; this second medium does not support growth of the cells. This feature distinguishes this invention over the invention described and claimed in our companion case (17894) filed concurrently herewith. As used hereinafter, the following terms have their meanings defined:

Osmotic Stabilizer : Any compound that, when added to the medium increases the osmolarity of the medium. These include, for example, electrolytes such as NaCl, sugars such as sorbitol, polyhydric alcohols such as glycerine etc.

mnn9 yeast: Strains of S. cerevisiae containing the mnn9 genotype which are described in part in U.S. Patent Application No. 202,764 corresponding to EP-A-0344864, and incorporated herein by reference.

Foreign DNA : Fragment(s) of DNA that encode a foreign (heterologous) or protein glycoprotein that is not produced naturally by yeast.

Heterologous Protein Production or Expression : Production of a non-yeast protein in yeast cells. The DNA required to produce this protein can be contained within one of the chromosomes of the yeast genome (integrated) or may be present extra-chromosomally on a plasmid vector.

Plasmid Vector : An extrachromosomal segment of DNA capable of autonomous replication in yeast that is able to accept other fragments of DNA that encode and/or direct the synthesis and expression of a heterologous protein.

Genome : The DNA within a cell that defines the characteristics of that cell.

Milliosmolarity (mOsm) : The chemical term used to describe the strength of the tendency of water to flow from a solution containing a higher concentration of solutes to a solution containing a lower concentration of solutes. The solutions of solutes can be separated from one another by any semi-permeable membrane, including, but not limited to, the membrane surrounding living cells. Yeast possess such a semi-permeable membrane.

Inducer : Chemical or environmental conditions that, when added to the medium under appropriate conditions, result in the synthesis of a heterologous protein. An example of an inducing compounds is galactose (GAL promoters). An example of inducing environmental conditions includes temperature shift ($\alpha$-mating factor promoter).

Broadly speaking, this invention comprises:

1) growing under suitable nutrient conditions, host cells containing a suitable fragment of DNA for the expression of a heterologous protein.

2) transferring the cells to a production medium in which the environmental conditions within the production medium are adjusted to maximize production of the heterologous protein and simplify isolation of the heterologous protein.

3) controlling the environmental conditions within a non-nutrient medium to maximize the production of heterologous proteins in hosts with mutations in structural components of cell walls and/or plasma membranes.

According to the present invention, the osmolarity of the medium is critical to the optimal production of both heterologous proteins in host strains with mutations in structural components of cell walls and/or plasma membranes (e.g. mnn9 ). The present invention also encompasses within its scope media for the production of heterologous proteins using other types of cells (e.g. mammalian cells, insect cells, bacteria, fungi) in non-growth media.

Other aspects and advantages of the invention will be apparent to those skilled in the art upon consideration of the following detailed description which provides illustrations of the practice of the invention in its presently preferred embodiments, without, however, limiting the same thereto.

## DETAILED DESCRIPTION OF THE INVENTION

In a typical practice of this invention, a host cell and a plasmid vector are used to produce various heterologous proteins. If the cells exhibit osmotic fragility, osmotic stabilization of the medium is required to minimize any growth advantage that wild-type host cells might have over mutant cells. Optimal cellular growth in mnn9 yeast occurs, for example, when the osmolarity of the medium in which the cells are present is between 300 and 700 mOsm. Non-growing cells will retain their genotypes. Cells can be removed from the growth medium which is high in contaminating proteins and be placed in an second, non-growth medium containing an osmotic stabilizer to prevent cell lysis, if necessary, and also containing appropriate compounds or conditions for the induction of heterologous protein production.

The osmolarity of the medium is determined using an Osmette A, a device manufactured by Precision Systems, Inc. of Sudbury, Massachusetts. The osmolarity of the sample, relative to standards of known osmolarity, is calculated based on a measurement of freezing-point depression according to the method of the manufacturer. In order to establish a model to evaluate media and process parameters, the middle protein of the Hepatitis B virus envelope (also called preS2 + S) was chosen as a model heterologous protein. As it is known in the art, the choice of the preS2 + S protein is biomedically relevant as this protein assembles into a form in the recombinant host cells which confers on it the properties of an effective immunogen as well as, potentially, an assay reagent since it displays important protective epitopes of the Hepatitis B virus. It is however obvious to those skilled in the art that any foreign reporter gene could have been chosen and as such could include, for example, the other envelope genes of the Hepatitis B virus. The production of heterologous protein is measured in crude cell lysates by radioimmunoassay (RIA) and immunoblot analysis using antibody specific for the particular protein product according to the methods described by Ellis et al . (1988. Preparation and testing of a recombinant-derived hepatitis B vaccine consisting of preS2 + S polypeptides in : Viral Hepatitis Liver Disease, A.J. Zuckerman (ed.), Alan R. Liss, New York, pp (1079-1086)). Cellular mass production is determined using either light scatter at a wavelength of 660 nm (absorbance, $A_{660}$), or by dry cell weight determinations. It is obvious to those skilled in the art that the selection of host strains of S. cerevisiae as a model extends to all species and cells derived from, but not limited to, mammals, insects, bacteria, and other fungi. Mutants which exhibit increased fragility include species and cells derived from the aforementioned hosts which may or may not contain mutations in cell wall or membrane biosynthesis.

The genus Saccharomyces is composed of a variety of species. S. cerevisiae is most commonly used as a host for the recombinant DNA-mediated expression of a variety of foreign polypeptides However, the distinctions among other species of the genus Saccharomyces are not always well-defined. Many of these species are capable of interbreeding with S. cerevisiae and are likely to possess promoters which are analogous or identical to promoters in S. cerevisiae . Therefore, it will be obvious to those skilled in the art that, for the expression of preS2 + S polypeptides, the selection of a host strain extends to other species of the genus Saccharomyces , including, but not limited, to carlsbergensis , diastaticus , elongisporus , kluyveri , montanus , norbensis , oviformia , rouxii , and uvarum .

Several yeast genera such as Candida , Hansenula , Pichia , and Torulopsis have been shown to contain similar metobolic pathways for the utilization of methanol as a sole carbon source for growth. The gene for alcohol oxidase, an enyzme which participates in this metabolic pathway, has been isolated from Pichia pastoris . The P. pastoris alcohol oxidase promoter has been isolated and shown to be susceptible to induction of expression by methanol. Such an inducible promoter is useful for the expression of polypeptides in yeast. In particular, this promoter has been shown to be active on a plasmid for the inducible expression of the S domain of the hepatitis B viral envelope in P. pastoris in particulate form. This observation highlights the ability of other yeast genera to function as hosts for the recombinant DNA-mediated expression of polypeptides in immunologically-active form. Therefore, it will be obvious to those skilled in the art that, for the expression of preS2 + S, the selection of a host strain extends to species from other genera of yeast from the Families Saccharomycetaceae and Cryptococcaceae , including but not limited Candida , Hansenula , Kluyveromyces , Pichia , Saccharomyceopsis , and Torulopsis .
The following examples serve to further illustrate the embodiments of the present invention:

## EXAMPLE 1

### Effect of Osmolarity of Buffered-Salts Solution on preS2 + S Productivity

Production of preS2 + S was directed from a plasmid vector containing the galactose-regulated GAL10 promoter, preS2 + S ORF, and ADH1 terminator (expression cassette) as described in Kniskern et al (1989) Proceedings of the Fifth International Symposium on Immunobiology of Proteins and Peptides.
A vial containing the frozen recombinant yeast was thawed and the contents were inoculated onto a plate of seed medium (5XLEU/HSA: 0.85% yeast nitrogen base without amino acids and ammonium sulfate/0.02% adenine/0.025% tyrosine/0.02% uracil/12.8% sorbitol/0.5% ammonium sulfate/4% glucose/0.0032% zinc sulfate/0.0038% ferric chloride/0.01% L-arginine/0.005% L-histidine-HCl/0.03% L-isoleucine/0.02% L-lysine-HCl/0.005% L-methionine/0.03% L-phenylalanine/0.02% L-tryptophan/2% agar).
The plate was incubated at 28° C for 3 days. The growth from the surface of the plate was inoculated into a 2-L flask containing 500 mL of seed medium (YEHD/HS: 2% yeast extract/1% soy peptone/2% glucose/21.8% sorbitol). The flask was incubated at 28° C and 350 RPM for 20 hours. The contents of the flask were then inoculated into a 16-L fermentor containing 11 L of YEHD/HS. The fermentor was operated

4

at 28°C, 500 RPM, and 5 L/minute air. After 11 hours incubation, the contents of the 16-L fermentor were transferred into a 250-L fermentor containing 200-L of production medium (2XYEHD/HS: 4% yeast extract/2% soy peptone/4% glucose/21.1% sorbitol). The fermentor was operated at 28°C, 260 RPM, and 90 L/minute air. After 16 hours incubation, a large aliquot of broth was removed from the fermentor and treated in the following manner:

A. One 50-mL aliquot of cells was transferred directly to a 250-mL shake flask and induced with 2% (w/v) galactose; this condition simulated batch induction and served as the control for the experiment.

B. Four other 50-mL aliquots were centrifuged and the supernatant decanted. Cells were resuspended in phosphate-buffered saline (PBS) to give a 4X cell concentrate; this concentration was chosen to mimic a concentration of cells that would be produced in a fed-batch fermentation. Two flasks containing a 4X cell concentrate were prepared. One flask was induced with 2% galactose (w/v) while the other was induced with 4% galactose (w/v). All of the flasks were incubated for an additional 34 hours at 350 RPM and 28°C. The data from this experiment are presented in Table 1. An initial absorbance reading of the concentrated cells was not determined.

The volumetric productivity (amount of preS2 + S per liter of fermentation broth) data are presented in two ways: the column designated "standard volumetric productivity" contains results of preS2 + S production directly from the flasks, which contained a 4X cell concentrate; the last column presents the volumetric productivity normalized for the cellular mass in the control flask. In other words, the volumetric productivity in the resting cell flasks was reduced by the ratio of cellular mass in the control flask to the resting cell flask. When the cells are induced after removing them from the growth medium, the non-normalized data indicate a 4.5-fold increase in volumetric productivity, from 1.5 mg preS2 + S/L to 7 mg preS2 + S/L. The normalized data indicate a 1.7-2.5-fold increase in volumetric productivity.

TABLE I

| Productivity of Cells Induced in a Nutrient-Deficient Medium | | | | | | |
|---|---|---|---|---|---|---|
| Condition | Cellular Yields Absorbance (600 nm) | PreS2 + S RIA (mg/L) | Protein (mg/mL) | Sp. Act. (μg/mg) | Volumetric Productivity | |
| | | | | | Standard (mg/L) | Normalized (mg/L) |
| Batch Fermentation (control) | 7.2 | 33.2 | 19.0 | 1.7 | 1.5 | 1.5 |
| Resting Cells + 2% Galactose | 12.8 | 57.2 | 10.0 | 5.7 | 4.6 | 2.6 |
| Resting Cells + 4% Galactose | 13.6 | 82.5 | 12.0 | 6.9 | 7.0 | 3.7 |

Specific activity also-increased between the cells induced in phosphate-buffered saline (PBS) and control cells, from 1.7 μg preS2 + S/mg protein to 5.7-6.9 μg preS2 + S/mg protein. Thus, cells induced in PBS exhibited a 3.5-4-fold increase in specific activity over the control cells.

This example illustrates an alternate method for growing the cells in the presence of lower sorbitol concentrations. Any reversion from mnn9 to MNN9 should occur only when cells are dividing. If the lower concentration of sorbitol in the seed and production medium affects the ratio of mnn9 to MNN9 cells due to growth rate differences between mnn9 and MNN9 cells or due to differences in reversion rates, this alternative process would enable the fermentation to be conducted with high sorbitol concentration. Then, prior to induction, the sorbitol and growth medium could be diafiltered away from the cells, galactose added, and the fermentation continued to the optimal harvest time. Another potential advantage of this process is that the amount of protein in the clarified cell extract is lower than that of the control cells, suggesting a product in the clarified cell extract that is enriched for product which may be easier to isolate.

EXAMPLE 2

Effect of Sorbitol on preS2 + S Productivity

The objective of this experiment was to determine the effects of sorbitol on preS2 + S productivity. Cells were induced in a non-growth medium to distinguish between the effects of sorbitol on cellular growth and the effects of sorbitol on preS2 + S productivity.

Cells from a 2-L flask grown in 2XYEHD/HS were separated from the fermentation medium by centrifugation, washed, and resuspended in 25 mL of phosphate buffer (PBS) with varying concentrations of sorbitol at twice the original cell concentration (Table 2).

Galactose was then added to the flasks at a final concentration of 2% (w/v). Incubation continued for an additional 49 hours at which time cells were prepared for preS2 + S RIA analysis.

TABLE 2

| | DATA FOR EXAMPLE 2 | | | | |
|---|---|---|---|---|---|
| MEDIUM # | CONCENTRATION OF SORBITOL IN PHOSPHATE-BUFFERED SALINE SOLUTION ( $\underline{M}$ ) | OSMOLARITY mOsm | CELLULAR YIELD ($A_{660\ nm}$) | | SPECIFIC ACTIVITY ($\mu$g preS2 + S/mg protein) |
| | | | initial/final | | |
| A | 0.0 | 201 | 10.8 | 7.8 | 6.0 |
| B | 0.1 | 301 | 10.8 | 9.0 | 8.7 |
| C | 0.25 | 450 | 10.8 | 8.0 | 7.8 |
| D | 0.5 | 700 | 10.8 | 8.0 | 7.6 |
| E | 0.75 | 955 | 10.8 | 7.2 | 3.5 |
| F | 1.0 | 1205 | 10.8 | 7.4 | 1.3 |
| G | 1.2 | 1400 | 10.8 | 9.6 | 0.6 |

The cells did not grow in RCM-8 with the various concentrations of sorbitol (Table 2). The cellular mass decreased in all of the flasks which contained the non-growth medium. No difference in the amount of decrease in cellular mass was observed in the flasks with various concentrations of sorbitol.

The results of this example are presented in Table 2.

When the concentration of sorbitol was above 0.5M, preS2 + S specific activity decreased. Activities of 7.6 $\mu$g/mg were observed when the sorbitol concentration was between 0.1 and 0.5M; the specific activity declined to 0.6 $\mu$g/mg when the sorbitol concentration was 1.2M.

**Claims**

1. A process for the production of heterologous proteins comprising:
   (a) growing host cells capable of expressing foreign proteins in a medium which will support the growth of said cell;
   (b) separating the cells from the growth medium, and
   (c) transferring the separated cells into a medium which will support the production of heterologous proteins.

2. A process according to Step (a) in Claim 1 in which the growth medium comprises assimilable sources of nitrogen, amino acids, carbon and trace elements.

3. A medium according to Claim 2 whose osmolarity is optimized for maximum growth for the host cell.

4 . A medium according to Step (c) in Claim 1 which comprises water and a suitable inducer.

5. A medium according to Step (c) in Claim 1 which comprises a buffer and a suitable inducer.

6 . A medium according to Step (c) in Claim 1 which comprises water.

7 . A medium according to Step (c) in Claim 1 which comprises a buffer.

8 . A medium according to Claims 4,5,6 or 7 which also contains an osmotic stabilizer for the maximum expression of a heterologous protein.

9 . A medium according to Claim 8 wherein the concentration of stabilizer is adjusted to achieve 301 to 700 mOsm.

10. A process according to Claim 1 wherein the host cells are a yeast or fungus.

11. A process according to Claim 10 wherein the host cell is a species of yeast derived from the Families Saccharomycetaceae or Cryptococcacese .

12. A process according to Claim 11 wherein the yeast species is from the genus Saccharomyces .

13 . A process according to Claim 12 wherein the yeast species is Saccharomyces cerevisiae .

14. A process for the production of heterologous proteins comprising cultivation of S . cerevisiae with a mutation or mutations in the biosynthesis of cell wall or plasma membrane components and an expression cassette suitable for production of the heterologous protein in one medium as defined in Claim 1 Step a and transferring the cells to a second medium as defined in Claim 1 Step c which is optimized for the production of the heterologous protein but which does not promote cellular growth.

15 . A process according to Claim 14 wherein the mutation is in a gene for glycoprotein biosynthesis.

16 . A process according to Claim 15 wherein the mutation is in the mnn9 gene.

17 . A process for the production of a heterologous protein in yeast cells with a mutation or mutations in the biosynthesis of cell walls or plasma membrane components, containing an expression cassette suitable for the production of a heterologous protein in a non-growth medium according to Step c of Claim 1.

18. A process according to Claim 17 wherein the mutation is in a gene for glycoprotein biosynthesis.

19. A process according to Claim 17 wherein the mutation in is the mnn9 gene.

20. A process for the production of preS2 + S comprising cultivation of cells of S . cerevisiae with the mnn9 genotype and a yeast DNA plasmid containing an expression cassette encoding the hepatitis B viral envelope protein is a suitable growth medium and transferring the cells to an osmotically stable nutrient deficient medium containing unducer, according to Claim 1.